Europäisches Patentamt

European Patent Office

Office européen des brevets

(19)

(11) Veröffentlichungsnummer: **0 095 034**

**B2**

(12) **NEUE EUROPÄISCHE PATENTSCHRIFT**

(45) Veröffentlichungstag der neuen Patentschrift:
23.01.91

(51) Int. Cl.⁵: **A 61 F 13/15**

(21) Anmeldenummer: **83103636.3**

(22) Anmeldetag: **14.04.83**

(54) Verfahren zur Herstellungeiner Wegwerfwindel.

(30) Priorität: **14.04.82 JP 61946/82**

(43) Veröffentlichungstag der Anmeldung:
**30.11.83 Patentblatt 83/48**

(45) Bekanntmachung des Hinweises auf die
Patenterteilung:
**24.07.85 Patenblatt 85/30**

(45) Bekanntmachung des Hinweises auf die
Entscheidung über den Einspruch:
**23.01.91 Patentblatt 91/04**

(84) Benannte Vertragsstaaten:
**BE CH LI LU NL**

(56) Entgegenhaltungen:
| DE-A-1 943 211 | GB-A-2 078 811 |
| DE-A-2 649 948 | US-A-3 860 003 |
| DE-A-2 817 998 | US-A-4 050 462 |
| FR-A-2 025 791 | US-A-4 081 301 |
| FR-A-2 063 794 | US-A-4 284 454 |
| FR-A-2 412 276 | US-A-4 300 562 |

(73) Patentinhaber: **UNI-CHARM CORPORATION
182, Shimobun Kinsei-cho ·
Kawanoe-shi Ehime-ken (JP)**

(72) Erfinder: **Suzuki, Migaku
2666 Kawanoe-cho
Kawanoe-shi Ehime-ken (JP)**
Erfinder: **Sasaki, Satoshi
385-1, Handaotsu Kaneda-cho
Kawanoe-shi Ehime-ken (JP)**
Erfinder: **Mitsuno, Takashi
385-1, Handaotsu Kaneda-cho
Kawanoe-shi Ehime-ken (JP)**
Erfinder: **Inagaki, Hiroyuki
385-1, Handaotsu Kaneda-cho
Kawanoe-shi Ehime-ken (JP)**

(74) Vertreter: **Sperling, Rüdiger, Dipl.-Ing. et al
Patentanwälte Dr.Ing.H. Finke Dipl.Ing.H. Bohr
Dipl.Ing.S. Staeger Dipl.Ing.Dipl.Wirtsch.Ing. R
Sperling Müllerstrasse 31
D-8000 München 5 (DE)**

EP 0 095 034 B2

Courier Press, Leamington Spa, England.

# EP 0 095 034 B2

**Beschreibung**

Die vorliegende Erfindung bezieht sich auf eine Wegwerfwindel und ein Verfahren zum Einarbeiten bzw. Einsetzen von elastichen Teilen in eine solche Windel. Insbesondere bezieht sich die vorliegende Erlindung auf den Aufbau von elastischen Teilen, die dazu dienen, die beiden Seitenklappen einer Wegwerfwindel vom Typ eines Windelhöchens um die Schenkel eines Kleinkindes anzupassen, und auf ein Verfahren zum Einsetzen solcher elasticscher Teile in eine solche Windel.

Elastische Teile, die dazu dienen, beide Seitenklappen einer Wegwerfwindel um die Oberschenkel eines Kleinkindes anzupassen, wurden bereits bei bekannten Wegwerf-Höschenwindeln zum Einsatz gebracht, wobei die typischste einer solchen Windel nach dem Stand der Technik in der US—PS Nr. 3860003 offenbart worden ist. Gewöhnlich wurde ein einziges Gummiband als elastisches Teil in jede Seitenklappe der Windel eingearbeitet. Windeln, in denen ein derartiges elastisches Teil, bestehend aus einem einzigen Gummiband, eingearbeitet worden ist, üben einen lokalen oder linearen hohen Druck um die Oberschenkel eines Kleinkindes aus, wobei lineare Druckstellen am Oberschenkel des Kindes zurückbleiben und manchmal Schmerzen für das Kind verursacht werden, das solche Wegwerfwindeln trägt, insbesondere, wenn es diese Windel für eine relativ lange Zeit trägt.

Um derartige Nachteile zu eliminieren, wurde bereits vorgeschlagen, als elastisches Teil ein Gummiband zu verwenden, das eine relativ grosse Breite aufweist. Die Verwendung von in dieser Weise geformtem Gummiband weist jedoch Nachteile auf, die nachfolgend näher erläutert werden.

Vom Blickpunkt der Herstelltechnik ist es schwierig, ein Gummiband zu erhalten, das wesentlich dünner ist als solches Band, was bereits als ein derartiges elastisches Teil verwendet worden ist, und es ist nahezu unmöglich, ein Gummiband gleichmässiger Dicke zu erhalten, auch wenn das Fertigen von extrem dünnem Gummiband möglich ist. Demzufolge wird ein konventionelles Gummiband, das als elastiches Teil verwendet wird, breiter geformt und in die Wegwerfwindel mit einem Dehnungsverhältnis eingearbeitet, das ähnlich ist demjenigen, das als koventionelles Gummiband verwendet wird, so dass der Druck um den Oberschenkel des Kleinkindes gross ist, da die Zugkraft zu hock ist. Um ein elastiches Teil zu erhalten, das eine Zugkraft besitzt, die bezüglich des Drucks auf den Oberschenkel des Kleinkindes als optimal zu bezeichnen ist, ist es daher notwendig, das Dehnungsverhältnis dieses elastischen Teils in dem Mass zu verringern, wie die Breite eines solchen elastischen Teils zunimmt. Dies würde zu einer Zunahme der Kosten führen, und zwar aufgrund einer zusätzlichen Materialmenge für das elastische Teil, die erforderlich ist, um die Breite zu vergrössern, wie auch aufgrund eines zusätzlichen Materials für das elastische Teil, um das Dehnungsverhältnis zu reduzieren. Eine derartige Kostenzunahme ist sehr nachteilig für eine Wegwerfwindel, die mit den geringsmöglichen Kosten hergestellt werden soll.

Es ist sicherlich möglich, diese Nachteile dadurch zu überwinden, wenn mehrere dünne Gummibänder als elastisches Teil verwendet werden, so wie es weiter unten in der Beschreibung der vorliegenden Erfindung näher erläutet wird. Jedoch wird man bei einer derartigen Massnahme mit Schwierigkeiten konfrontiert, die darin liegen, dass die Wegwerfwindel ein Massenprodukt mit niedrigen Kosten ist und die Gummibänder oder-fäden mittels Anklebens auf der unteren Lage bestehend aus einer Plastikfolie oder ähnlichem und/oder an der oberen Lage befestigt werden müssen, welche letztere aus einem Faservlies oder ähnlichem besteht, wobei beide Lagen Teile der Wegwerfwindel bilden. Desweiteren können diese Nachteile wirksam nur dann überwunden werden, wenn die als elastiches Teil verwendeten Gummifäden wesentlich dünner sind als das Gummiband konventioneller Art, wie es bisher bei den Wegwerfwindeln verwendet worden war. Dem steht jedoch gegenüber, dass, je feiner die Gummifäden sind, die als elastisches Teil Verwendung finden, und, je grösser die Anzahl dieser Gummifäden ist, das Handhaben dieser Gummifäden umso schwieriger ist zum Befestigen an der oder den genannten Lagen mittels Klebstoff, und die Funktionswirksamkeit ist erheblich reduziert.

Die Aufgabe der vorleigenden Erfindung besteht darin, ein verbessertes Verfahren zum Einlegen dieser Vielzahl von Gummifäden, die als elastische Teile in den Zugeordneten Komponenten der Wegwerfwindeln dienen, vorzuschlagen, wobei das Verfahren die Verfahrensschritte eines kontinuierlichen Zuführens dieser Gummifäden beinhaltet, insbesondere solcher in Form von jeweils einzelnen Gummibändern, die durch schienbare Seite an Seite angeordnete Verbindungen gehalten werden, wobei jede eine innere Trennbarkeit aufweist, und wobei die Zuführung zu den einzelnen Komponenten vorgesehen ist, während die Bänder sauber in eine vorbestimmte Anzahl von Gummifäden getrennt werden und anschliessend diese Gummifäden in den Komponenten mittels eines Klebemittels befestigt werden, so dass das gewünschte Einsetzen dieser Gummifäden in die Wegwerfwindel bei hoher Wirksamkeit erzielt wird.

Weitere Aufgabe der Erfindung ergeben sich aus der nachfoldenden Beschreibung.

Die Aufgabe der Erfindung wird bei einem Verfahren zum Einarbeiten bzw. Einlegen von elatischen Teilen in eine Wegwerfwindel bestehend aus einer oberen und einer unteren Lage und einem zwischen diesen beiden Lagen befestigten Körper mit absorbierenden Eigenschaften und mit jeweils durch sich über beide Seiten der einander gegenüberliegenden Außenränder des Absorbierkörpers hinauserstreckende Seitenklappen, zwischen denen jeweils mindestens drei in geeigneten Abständen parallel zueinander über einen Breitenbereich von 10 mm bis 35 mm hinweg Gummifäden gehalten sind, dadurch gelöst, daß folgende Verfahrensschritte durchgeführt werden:

Zuführen eines elastischen Bandes, bestehend aus sechs (6) bis neunzig (90) Gummifäden, die parallel verlaufend miteinander verbunden sind, wobei die Gummifäden jeweils im wesentlichen einen Querschnitt

2

von 0,03 mm² bis 0,45 mm² und einen Gesamtquerschnitt von 0,18 mm² bis 2,7 mm² aufweisen,

Trennen der Gummifäden entlang ihrer Verbindungen in einzelne Gummifäden, während sie in einem Verhältnis von 100% bis 400% gedehnt werden,

Zuführen von jeweils drei (3) bis fünfundvierzig (45) Gummifäden der sechs (6) bis neunzig (90) Gummifäden zu Bereichen der Windel hin, um in die Seitenklappen in geeigneten Abständen eingeformt zu werden,

Aufbringen eines adhäsiven Agens auf die Gummifäden,

Ankleben dieser Gummifäden in gedehnten Zustand in den oben bezeichneten Bereichen unmittelbar auf den jeweiligen Lagen in den Seitenklappen.

Im folgenden werden bevorzugte Ausführungsbeispiele der vorliegenden Erfindung anhand der beigefügten Zeichnungen näher beschrieben. Es zeigen:

Fig. 1 eine ebene Abwichlung zum Teil mit herausgebrochenen Bereichen einer Wegwerfwindel mit dem erfindungsgemässen Aufbau,

Fig. 2 einen Schnitt entlang der Linie II—II in Fig. 1,

Fig. 3 A und B eine perspektivische Teilansicht, die allgemein die elastischen Teile mit besonderen Querschnittsbereichen zeigt, die im Erfindungsbereich der vorliegenden Erfindung liegen,

Fig. 4 eine perspektivische Teilansicht eines Ausführungsbeispiels eines elastischen Teils, wie es zum Ausführen des erfindungsgemässen Verfahrens verwendet werden kann,

Fig. 5 eine schematische teilweise Seitenansicht einer Vorrichtung zum Herstellen der erfindungsgemässen Wegwerfwindel,

Fig. 6 eine Ansicht von oben auf eine Teilstation des elastischen Teils in der Vorrichtung gemäss Fig. 5,

Fig. 7 eine perspektivische schematische Ansicht einer mechanischen Station zum klebenden Befestigen der elastischen Teile an der Windel,

Fig. 8 eine perspektivische Ansicht einer Düse, die zum Aufbringen des Klebmittels auf die elastischen Teile dient,

Fig. 9 eine Explosionsdarstellung der Düse aus Fig. 8,

Fig. 10 einen Schnitt durch eine Mittellinie der Düse gemäss Fig. 8, und

Fig. 11 und 12 Diagramme mit charakteristischen Kurven, die jeweils entstehen, wenn ein einzelnes Gummiband und wenn eine Gruppe von getrennten Gummifäden als das elastische Teil verwendet werden.

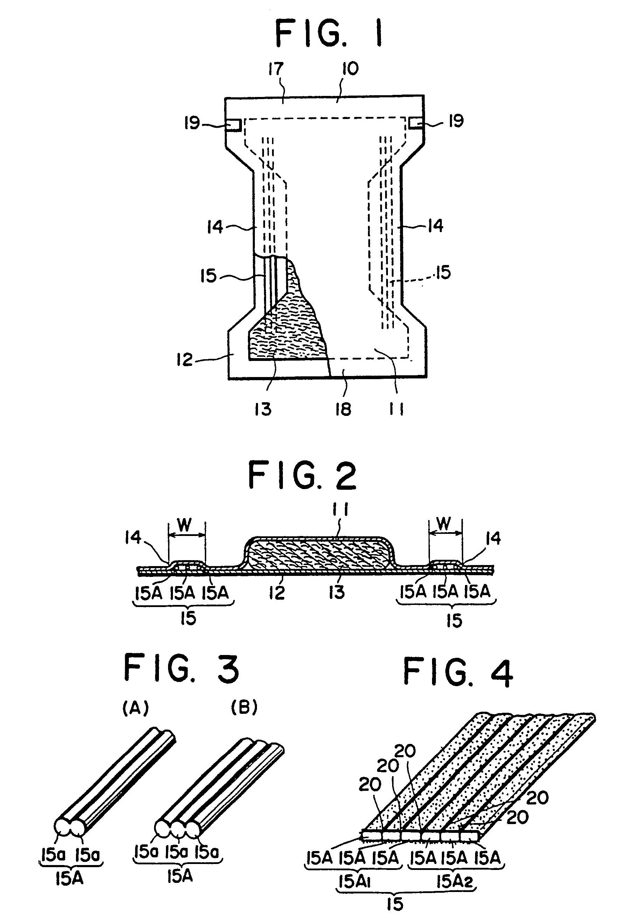

Es wird zunächst auf die Fig. 1 und 2 Bezug genommen. Eine Wegwerfindel 10 weist die folgenden Elemente auf: eine wasserdruchlässige obere Lage 11 aus Faservlies oder ähnlichem, eine wasserdurchlässige untere Lage 12 aus einem Plastikfilm oder ähnlichem, ein elastisches Teil 15, das in den jeweiligen Seitenklappen 14 anmgeordnet ist, welche Seitenklappen von Bereichen beider Lagen 11 und 12 gebildet werden, die sich in gegenüberliegende Richtungen über die Aussenränder des Absorbierkörpers 13 erstrecken und dicht aufeinander zu liegen, so dass das elastische Teil 15 in Längsrichtung elastisch dehnbar ist. Eine derartige Windel 10 ist, wenn sie einem Kleinkind angelegt wird, mit druckempfindlichen Verbindungs-Befestigungsbändern ausgestattet, welche vorher von gegenüberliegenden Enden eines Hüftbereichs 17 zu entsprechenden gegenüberliegenden Enden des anderen Hüftbereichs 18 angebracht worden sind. Die elastischen Teile 15 weisen jeweils eine Vielzahl von Gummifäden auf, was im weiteren Verlauf näher im Detail beschrieben wird, und wobei die Wegwerfwindel allgemein den Aufbau aufweist, wie eine solche, in der die elastischen Teile aus einem einzigen Gummiband bestehen, wie es zum Beispiel aus der US—PS Nr. 3860003 bekannt ist.

Obwohl in den Fig. 1 und 2 eine Wegwerfwindel 10 dargestellt ist, deren elastische Teile 15 jeweils parallel angeordnet drei Gummifäden 15A in den jeweiligen Seitenklappen 14 aufweisen, ist es auch möglich, innerhalb des Schutzbereichs dieser Erfindung elastische Teile 15 zu verwenden, die drei (3) bis funfundvierzig (45) Gummifäden aufweisen, die jeweils eine Querschnittsfläche von 0,03 mm bis 0,45 mm² und eine Gesamtquerschnittsfläche von 0,09 bis 1,35 mm² aufweisen, welche Gummifäden in den Seitenklappen 14 so angeordnet sind, dass sie jeweils einen parallelen Abstand aufweisen. Diese Gummifäden 15A können eine gleichförmige Querschnittsfläche aufweisen, oder en können in manchen Fällen bestimmte Fäden unter diesen einen unterscheidlichen Querschnitt besitzen. Im letzteren Fall wird es sinnvoll sein, die Gummifäden 15A mit einem relativ geringen Querschnitt an der Innenseite innerhalb der jeweiligen Seitenklappen 14 und die Gummifäden 15A mit dem relativ grossen Querschnitt an der Aussenseite in den jeweiligen Seitenklappen 14 anzuordnen.

Die elastischen Teile 15 des ersteren Typs werden vorzugsweise zum Ausführen des erfindungsgemässen Verfahrens verwendet, was später näher beschreiben wird. In der erfindungsgemässen Wegwerfwindel 10, bei der jeweils elastische Teile 15 drei (3) bis fünfundvierzig (45) Gummifäden 15A in den jeweiligen Seitenklappen 14 vorgesehen sind, werden in den jeweiligen Seitenklappen 14 drei (3) bis fünfundvierzig (45) elastisch dehnbare Linien hergestellt, welche dazu dienen, die Seitenklappen um die Oberschenkel des Kleinkindes herum anzupassen. Die jeweiligen Gummifäden 15A können einen kreisförmigen, einen elliptischen oder einen quadratischen Querschnitt aufweisen. Desweiteren kann eine Vielzahl von Gummifadenelementen 15a integral Seite an Seite parallel angeordnet und miteinander verbunden sein, um jeden Gummifaden 15A zu bilden, der einen kombinierten Querschnitt aufweist, wie er durch die Fig. 3A oder 3B dargestellt ist. Die Gummifäden 15A, welche jeweils einen solchen kombinierten Querschnitt entsprechend einer Vielzahl von Kreisen aufweisen, die aneinander angeordnet sind, kann beispielsweise dadurch erhalten werden, dass die jeweiligen Gummifadenelemente 15a gegossen und anschliessend, bevor sie ausreichend abgekühlt sind um sich zu verfestigen, zusammengeschmolzen werden.

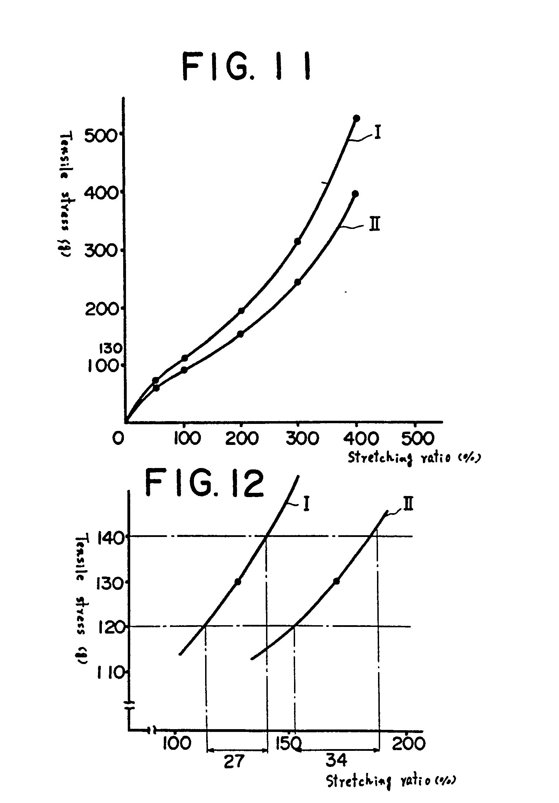

Wenn eine Vielzahl von Gummifäden 15A als ein elastisches Teil 15 verwendet werden, dann ist die Zugkraft reduziert und das Dehnungsverhältnis im Verhältnis zu einem einzelnen Gummiband, das als das elastische Teil verwendet wird, erhöht, selbst wenn die Gesamtquerschnittsfläche, das Material und das Dehnungsverhältnis der Gummifäden 15A identisch ist zu demjenigen des Einzelgummibandes. Selbst der tolerierbare Bereich eines ungleichmässigen Dehnungsverhältnisses wird vergrössert, wenn eine derartige Vielzahl von Gummifäden 15A als elastisches Teil 15 verwendet werden. Dies kann man leicht anhand der Fig. 11 und 12 verstehen, welche das Ergebnis der Experimente der Erfinder darstellen.

In Fig. 11 zeigt das Diagramm charakteristische Kurven, welche von den Kurven I und II im Vergleich erhalten worden sind, wenn diese Proben einer Zugkraft g, auf der Koordinate aufgetragen, bei einem Dehnungsverhältnis (%), auf der Abszisse aufgetragen, ausgesetzt worden sind. Die Probe I besteht aus einem einzelnen Gummiband aus Naturgummi, das eine Länge von 100 mm, eine Breite von 6,6 mm und eine Dicke von 0,2 mm aufweist, wohingegen die Probe II aus drei getrennten Gummibändern besteht, die heweils eine gleiche Dicke von 3,2 mm besitzen, um eine Zugkraft von 130 g in jedem elastischen Teil hervorzurufen. Es wurde herausgefunden, dass eine solche Zugkraft einen optimalen Druck um den Oberschenkel des Kleinkindes hervorruft, kann die Probe I beispielsweise kaum mehr als annähernd um 120% gedehnt werden, wohingehend die Probe II leicht bis annähernd 170% gedehnt werden kann.

In Fig. 12 ein Diagramm dargestellt, das eine Vergrösserung eines Teils des Diagramms aus Fig. 11 enthält, und den tolerierbaren Bereich eines ungleichen Dehnungsverhältnisses für jeweils die Probe I und die Probe II betrifft, wenn diese Proben in die zugeordneten Windeln eingearbeitet sind und einer Zugkraft von 130 g ± 10 g als Erfordernis für die Windelauslegung ausgesetzt sind. Betreffs des tolerierbaren Bereichs eines ungleichmässigen Dehnungsverhältnisses zeigt das experimentelle Ergebnis annähernd 27% für die Probe I und 34% für die Probe II. Der tolerierbare Bereich eines ungleichmässigen Dehnungsverhältnisses ist für die Probe II daher grösser als für die Probe I, so dass eine solche Konstruktion der Probe II eine Zugbefestigung des elastischen Teils in einem Schritt der Ziehbehandlung während der Durchführung des Verfahrens gemäss der Erfindung erleichtert.

Wie aus den Ergebnissen der von den Erfindern durchgeführten Experimente zu erkennen ist, erlaubt die Verwendung einer Vielzahl von Gummifäden 15A als elastisches Teil 15 entsprechend einem einzigen Gummiband, geteilt in getrennte Gummibänder, ein Dehnungsverhältnis, das höher ist als in dem Fall, in dem das einzelne Gummiband als elastisches Teil verwendet wird, um einen gewünschten Druck um den Oberschenkel des Kleinkindes zu erzielen, da die Zugkraft im ersten Fall geringer ist als im letzteren. Mit anderen Worten, die Länge des elastischen Teils 15 kann reduziert werden, was sehr wirtschaftlich ist. Die Folge davon ist, dass bei einer erfindungsmässen Wegwerfwindel, bei der eine Vielzahl von Gummifäden 15A als elastisches Teil 15 verwendet werden, die Materialmenge, die für das elastische Teil 15 notwendig ist, im Verhältnis zu Wegwerfwindel nach dem Stand der Technik reduziert werden kann, bei welchem Stand der Technik gewöhnlicherweise als elastisches Teil 15 ein einziges Gummiband verwendet wird. Somit kann erfindungsgemäss jede Wegwerfwindel mit entsprechend reduzierten Kosten hergestellt werden.

Die Gummifäden 15A, die als elastisches Teil dienen, weisen eine geringere Zugspannung auf, je mehr ihre Anzahl zunimmt, so dass ein entsprechendes höheres Dehnungsverhältnis angewendet werden kann, um diesen Vorteil noch zu erhöhen. Von den Erfindern durchgeführte Studien haben gezeigt, dass die Zugspannung bei elastischen Teilen, welche drei (3) bis fünfundvierzig (45) Naturgummifäden mit einer Einzelquerschnittsfläche von 0,03 bis 0,45 mm² und einer Gesamtquerschnittsfläche von 0,09 bis 1,35 mm² stark ansteigt, wenn das Dehnungsverhältnis 400% überschreitet mit dem Ergebnis, dass klar ausgeprägte Druckstellen an dem Oberschenkel des Kleinkindes zurückbleiben.

Dementsprechend werden die jeweiligen elastischen Teile 15 der erfindungsgemässen Windel einem Zugeffekt unterworfen, wenn diese elastischen Teile in die Windel eingearbeitet sind, so dass ein Dehnungsverhältnis von 100 bis 400%, vorzugsweise 120 bis 350%, insbesondere zwischen 250 und 300% erzielt wird.

Mit einem Dehnungsverhältnis, das geringer ist als 100%, würde die Gummimaterialmenge unwirtschaftlich erhöht.

Es wird darauf hingewiesen, dass ein einzelnes Gummiband, z.B. 0,2 mm dick und 6,6 mm breit, gewöhnlich als ein derartiges elatisches Band Verwendung gefunden hat, wobei dieses einzelne Gummiband in der Regel einem Zugeffekt mit einem Dehnungsverhältnis von 70 bis 100% ausgesetzt worden ist, wenn ein solches Gummiband in jede Seitenklappe der Winkel eingearbeitet worden war. Der Ausdruck «Dehnungsverhältnis von 100%» wird hier dazu verwendet, den Fall anzuzeigen, in welchem das elastische Teil von einer Anfangslänge von 100 mm auf eine Länge von 200 mm gedehnt wird.

In der erfindungsgemäss ausgestalteten Wegwerfwindel wird eine Vielzahl von Gummifäden 15A von jeweils relativ geringer Querschnittsfläche als das elastische Teil 15 in paralleler Anordnung und seitlichem Abstand eingesetzt, so dass die scheinbare Breite der jeweiligen elastischen Teile in den zugeordneten Seitenklappen (in Fig. 2 mit dem Bezugszeichen W bezeichnet) im Vergleich zu dem Fall vergrössert ist, bei welchem ein einziges Gummiband als elastisches Teil in der Wegwerfwindel nach dem Stand der Technik verwendent wird, so dass der Druck um den Oberschenkel des Kleinkindes entspreachend reduziert ist. Als Ergebnis werden durch die erfindungsgemässe Wegwerfwindel der Schmerz, den das Kleinkind oft durch das Tragen von Wegwerfwindeln nach dem Stand der Technik erleidet, wie auch die als Ergebnis eines solchen Tragens auf dem Oberschenkel des Kleinkindes auftretenden Druckstellen vermieden. Um solche Ergebnisse und Wirkungen zu erzielen, werden, obwohl dies mehr oder weniger auch von Charakteristika, wie beispielsweise der Steifigkeit der oberen Lage 11, der unteren Lage 12 und des Absorbierkörpers 13 alks Komponenten der Wegwerfwindel anhängt, eine Vielzahl von Gummifäden, die als die jeweiligen elastischen Teile 15 verwendet werden, in Bereichen von vorzugsweise 10 mm bis 35 mm, insbesondere zwischen 15 mm und 20 mm Breite ausserhalb von den jeweiligen Aussenrändern des Absorbierkörpers angeordnet.

Während des Gebrauchs der erfindungsgemäss aufgebauten Wegwerfwindel agieren die elastischen Teile 15, die jeweils eine Vielzahl von Gummifäden 15A aufweisen, so, als wenn diese elastischen Teile 15 jeweils integrale Teile wären, jedoch sind sie in Wirklichkeit jeweils in individuelle Gummifäden 15A geteilt, so dass diese Gummifäden 15A unabhängig voneinander gedehtnt werden und sich zusammenziehen, um das gewünschte Anpassen um des Kleinkinds Oberschenkel zu erleichtern. Die Tatsache, dass die elastischen Teile 15 in eine Vielzahl von Gummifäden 15A getrennt sind, ist vorteilhaft, um eine gute Luftdurchlässigkeit in den Bereichen dieser elastischen Teile 15 zu erhalten.

In Fig. 4 ist eine Ausführung von elastischen Teilen 15 dargestellt, die bei der Herstellung des erfindungsgemässen Verfahrens Verwendung findet. Das elastische Teil 15 weist sechs Gummifäden 15A auf, die im wesentlichen den gleichen Querschnitt aufweisen; die Gummifäden 15A werden in Form eines Einzelbandes durch provisorische Seite-an-Seite-Verbinder gehalten, weisen jedoch eine innere Fähigkeit auf, sich voneinander zu trennen.

Derartige Vebinder 20 der Gummifäden 15A bestehen aus linien einer schwachen Schmelzbindung oder aus provisorischer Verbindung, welche durch ein Sprühpuder, wie beispielsweise Talk, bewirkt werden kann, was auf die einzelnen Gummifäden aufgesprüht wird, sofort nachdem diese Gummifäden gegossen worden sind, um zu verhindern, dass sie an anderen Objekten kleben bleiben; diese Gummifäden werden dann in leichten Kontakt mineinander gebracht.

Dieser Puder, der an den jeweiligen Gummifäden 15A anhafter, kann, wenn notwendig, bei aktuellem Gebrauch von diesen Gummifäden entfernt werden. Desweiteren kann diese provisorische Verbindung 20 leicht durch eine leichte seitliche Zugkraft, die auf die Gummifäden aufgebracht wird, entfernt werden, so dass diese Fäden sauber entlang der provisorischen Verbindung 20 trennbar sind. Eine solche provisorisch zusammengefügte Anordnung von sechs (6) bis neunzig (90) individueller Gummifädern 15A kann als elastisches Teil 15 verwendet werden, da drei (3) bis fünfundvierzig (45) Gummifäden 15A auf jede der Seitenklappen 14 der erfindungsgemässen Windel, wie sie vorher beschreiben worden ist, aufgebracht und eingearbeitet werden. Die Anordnung solcher provisorisch miteinander verbundener Gummifäden wird von der Firma Rondex Co., Ltd. Kohoku-Ku, Yokohama, hergestellt und unter dem Handelsnamen «Rondex» vertrieben.

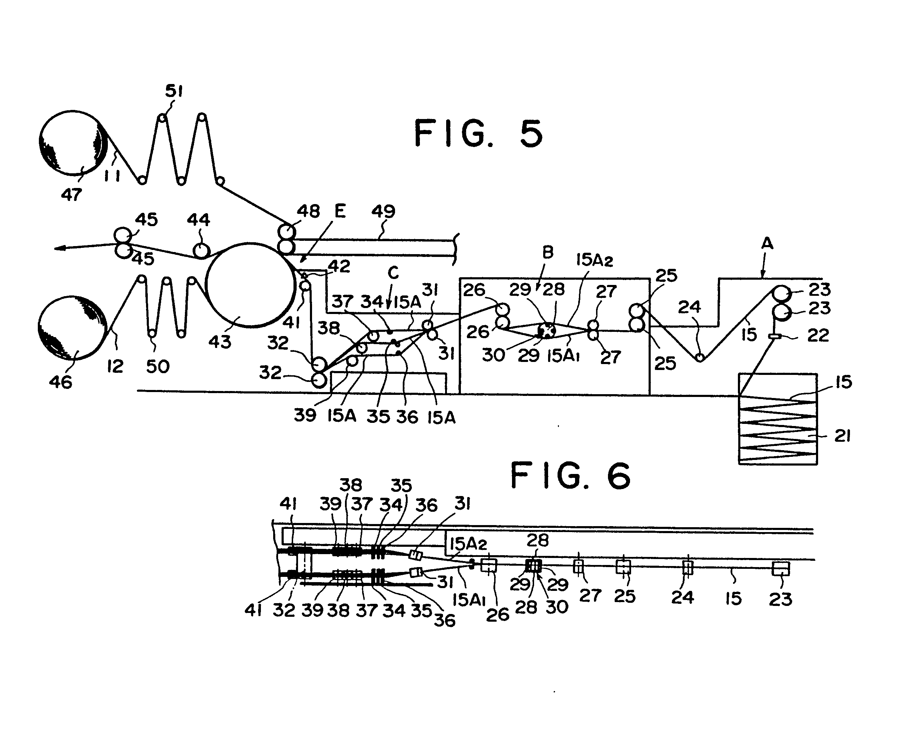

In den Fig. 5 bis 7 ist schematisch eine Vorrichtung zum Durchführen des erfindungsgemässen Verfahrens dargestellt. Diese Vorrichtung umfasst eine Zuführstation A, eine Ziehstation B, eine Trennstation C und für die elastischen Teile 15 jeweils eine Aufbringstation E.

Die Zuführstation A weist für die elastischen Teile 15 einen Vorratsbehälter 21, einen Führungsring 22 und ein Paar Führungswalzen 23, 24 auf, wobei die elastischen Teile 15 von der Zuführstation A zur Ziehstation B geleitet werden.

Die Ziestation B umfasst ein Paar Druckwalzen 25, 25, ein weiteres Paar Druckwalzen 26, 26, wobei die Umfangsgeschwindigkeit letzterer Walzen grösser ist als diejenige der ersten Druckwalzen, ein Paar Reinigungwalzen 27, 27 und eine Schlag- oder Rüttelwalze 30, wobei die letzten beiden Komponenten zwischen dem ersten und dem zweiten Paar Druckwalzen 25, 25 und 26, 26 angeordnet sind. Die Schlag- oder Rütterwalze 30 ist mit verschiedenen Stangen 23 versehen, die sich zwischen einander abgewandten Endplatten 28 erstrecken, an welchen die jeweiligen Enden der Stange 29 entlang des Umfangs der jeweiligen Endplatten 28 über den Umfang in gleichen Abständen sich erstreckend befestigt sind. Jedes elastische Teil ist einem Zugeffekt bein einem gewünschten Dehnungsverhältnis zwischen den beiden Druckrollenpaaren 25, 25 und 26, 26 ausgesetzt, während das vorher erwähnte Puder, das an dem elastischen Teil 15 anhaftet, mittels des Reinigungswalzenpaars 27 entfernt und das elastische Teil 15 des weiteren mittels der Schlag- oder Rüttelwalzen 30 gerüttelt oder geschlagen wird.

Die Menge Puder, die auf diese Weise von den Reinigungswalzen von dem elastischen Teil 15 abgestrichen und entfernt wird, wird durch Saugmittel oder ähnliches aufgefangen, welche Mittel in der Hähe dieser Säuberungsbürsten 27 angeordnet sind, das aufgefangene Puder wieder anschliessend in einen Behälter weitergeleitet (nicht dargestellt).

Obwohl das elastische Teil, das mittels einer Vorrichtung, wie sie in den Fig. 5 bis 7 dargestellt ist, behandelt werden kann, ist es nicht auf einen gleichen Typ beschränkt, wie er in Fig. 4 dargestellt ist. Es wird lediglich auf ein elastisches Teil der Ausbildung, wie sie in Fig. 4 dargestellt ist, Bezug genommen, um die Erfindung besser erklären zu können.

Das elastische Teil, das sechs (6) Gummifäden 15A aufweist, die provisorisch parallel in der vorher erwähnten Art miteinander verbunden sind, wird nun in zwei Blöcke geteilt, nämlich einen oberen Block $15A_2$ und einen unteren Block $15A_1$, welche jeweils an einer oberen und unteren Seite der Schlagwalze 30 vorbeigeführt werden, welche Schlagwalze somit zwischen den beiden Blöcken $15A_1$ und $15A_2$ angeordnet ist.

Das elastische Teil 15 wird von dem zweiten Paar Druckwalzen 26, 26 gezogen, wenn es entlang dieser Schlagwalze 30 geführt und dabei sukzessive in die jeweiligen Blöcke 15A₁, 15A₂ geteilt wird, Gleichzeitig dient die Schlagwalze 30 dazu, diese Blöcke 15A₁, 15A₂ zu schütteln oder zu schlagen, so dass diese Blöcke leicht in der Trennstation C in weitere individuelle Gummifäden 15A getrennt werden können, in welche Trennstation C die Blöcke eingeführt werden.

Die Trennstation C besitzt ein Paar Druckrollen 31, 31, ein weiteres Paar Druckrollen 32, 32, Führungsstangen 34, 35, 36, die auf vershiedenen Ebenen angeordnet sind und Führungswalzen 37, 38, 39, die den Führungsstangen 34, 35, 36 jeweils zugeordnet sind, wobei die letzteren Elemente zwischen den Druckwalzen 31, 31 und 32, 32 angeordnet sind. Die jeweiligen Blöcke 15A₁, 15A₂ werden in Einzelstränge 15A getrennt. Der erste Gummifaden 15A jedes Blocks wird gegen die Führungsstange 34 und dann gegen die obere Seite der Führungswalze 37 zur Anlage gebracht, der zweite Gummifaden 15A gegen die Führungsstange 35 und dann gegen die obere Seite der Führungswalze 38 und der dritte Gummifaden 15A gegen die Führungsstange 36 und die Oberseite der Führungswalze 39. Die jeweiligen Blöcke 15A₁ und 15A₂, die insoweit auf die zugeordneten Führungsstangen und Führungswalzen verteilt sind, werden zwischen die Zugwalzenpaare 32, 32 geführt, wobei die Blöcke in ihrem gedehnten Zustand gehalten und dabei sukzessive in die individuellen Gummifäden 15A durch die Führungswalze 34, 35 und 36 und den Führungswalzen 37, 38, 39 geteilt werden, wie bereits im Zusammenhang mit dem Zugeffekt erwähnt. So werden die individuellen Gummifäden 15A in die Auftragsstation E eingebracht.

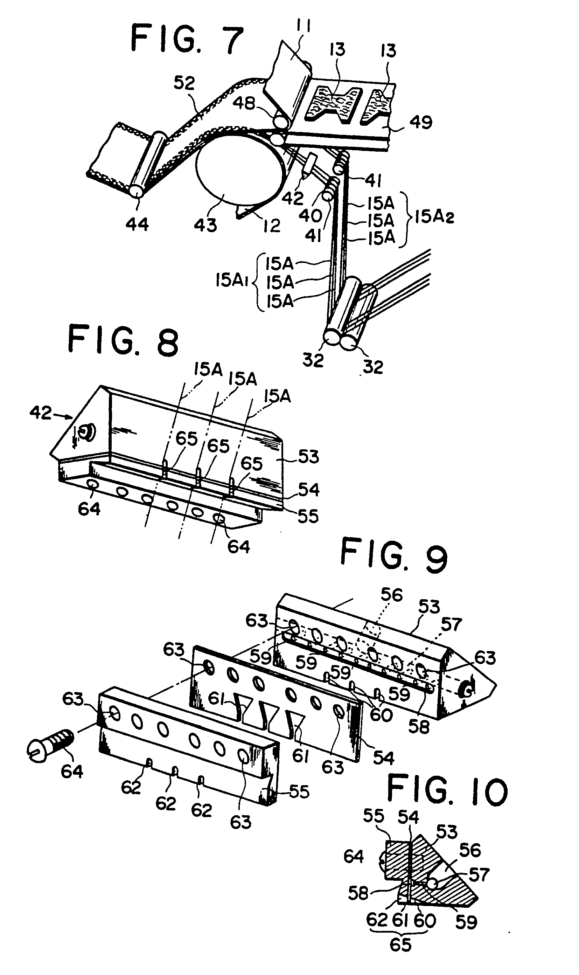

Die Auftragsstation E umfasst Führungswalzen 41, die an den Umfangsflächen mit einer Vielzahl von Nuten 40 (Fig. 7) versehen sind, Düsenmittel 42 und eine Abschreckwalze 43.

In der Nähe der Auftragsstation E sind eine Presswalze 44, ein Paar Druckwalzen 45, 45, eine Anlieferwalze 46, die dazu dient, den Plastikfilm 12 für die untere Lage der Windel zu tragen, welche um die Walze herumführt, eine Anlieferwalze 47 zum Tragen des Faservlies 11 für die obere Lage der Windel, welche Faservlieslage umn die Walze herumführt, eine Führungswalze 48, ein Förderband 49 zum Zuführen des Absorbierkörpers 13 der Windel, Führungsrollen 50, 51 und andere Mittel vorgesehen. Die einzelnen Gummifäden 15A werden durch die zugeordneten Führungswalzen 41 entlang der Nuten 40, welche um diese Walze herum ausgebildet sind, geführt und laufen an den zugeordneten Düsenmitteln 22 vorbei, mittels welcher die einzelnen Gummifäden 15A mit einem Adhesiv heiss schmelzenden Typs versehen und gleichzeitig bezüglich der Abstände, in denen die einzelnen Gummifäden 15A auf den zugeordneten Seitenklappen der Windel anzuordnen sind eingerichtet werden.

Die einzelnen Gummifäden 15A werden, während sie in gestrecktem Zustand gehalten werden, auf die obere Fläche des Plastikfilms 12 entlang der Seitenbereiche aufgeklebt. Der Plastikfilm 12 wird kontinuierlich auf die obere Seite der Abschreckwalze 43 zugeführt, und dient dazu, sowohl das Adhesiv abzuschrecken als auch zu festigen. Wie aus Fig. 7 erkennbar ist, wird das Faservlies 11 mittels der Führungswalze 48 auf die oberen Seite des genannten Plastikfilms 12 zugeführt, während der absorbierende Körper 13, der im Voraus geformt worden ist, über das Förderband 49 in Längsrichtung in regelmässigen Abständen in einen Spalt zwischen dem Faservlies 11 und dem Plastikfilm 12 gefördert wird, um zwischen diesen beiden Lagen gehalten zu werden. Das Faservlies 11 und der Plastikfilm 12 werden aus separaten Düsenmitteln (nicht dargestellt), die an gewünschen Positionen angeordnet sind, mit einem Adhesiv heiss schmelzenden Typs an ihren jeweiligen Innenseiten versehen, woraufhin diese beiden Teile aufeinander und miteinander verbunden werden, so dass der Absorbierkörper 13 in regelmässigen Abständen, wie oben erwähnt, festgelegt wird. Anschliessend werden kontinuierlich Windelstücke 52 durch die Pressrolle 44 gepresst und einem nachfolgenden Prozess angeliefert, in welchem diese kontinuierlichen Windeln 52 in Einzelwindel 10 geschnitten werden, wie sie in Fig. 1 dargestellt ist.

Fig. 8 bis 10 zeigt ein Ausführungsbeispiel der Düsenmittel 42. Die Dusenmittel 42 umfassen ein dreieckiges prismenförmiges erstes Teil 53, ein plattenförmiges zweites Teil 54 und ein Plattenförmiges drittes Teil 55. Das erste Teil 53 umfasst eine Einlassborung 56, die sich zur Mitte einer Seitenwand hin öffnet, einen Verteilkanal 57, der mit dieser Einlassbohrung 56 kommuniziert und sich in Längsrichtung des ersten Teils 53 erstreckt, eine Vielzahl von Düsenöffnungen 59, welche mit dem Verteilkanal 57 kommunizieren und in regelmässigen Abständen in eine längliche Ausnehmung 58 einmünden, die in Längsrichtung in der anderen Seitenwand ausgebildet ist, und eine Vielzahl von in regelmässigen Abständen in einem Ende des ersten Teils 53 ausgeformten Schlitzen 60. Das zweite Teil 54 umfasst eine Vielzahl von dreieckigen Schlitzen 61, die den gleichen Abstand aufweisen, wie die vorerwähnten Schlitze 60, an dem einen Ende des zweiten Teils 54 angeordnet. Das dritte Teil 55 umfasst gleichfalls eine Vielzahl von Schlitzen 62, die in den gleichen Abständen wie jene Schlitze 60 in dem einen Ende des dritten Teils 55 ausgebildet sind. Das erste Teil 53, das zweite Teil 54 und das dritte Teil 55 sind zu einem kompletten Düsenmittel 42 vereinigt, wenn Schrauben 64 in zugeordnete Gewindelöcher eingeschraubt werden, die in diesen drei Teilen vorgesehen sind. Die Düsenmittel 42 dieser Bauart sind in einer Bahn der drei Gummifäden 15A, die als jedes elastische Teil 15 dienen, zwischen der genannten Führungswalze 41 und der Abschreckwalze 43 eingesetzt, wie es in Fig. 5 erkannbar ist, so das Schlitze 65, die jeweils die genannten kleineren Schlitze 60, 61 und 62 enthalten, nach unten gerichtet sind. Ein nicht dargestellter Behälter ist angepasst, um das Auftragen, Blockieren und Bemessen des heiss schmelzenden Adhesivs zu steuern und ist mit der Einlassbohrung 56 verbunden. Wenn die jeweiligen drei Gummifäden 15A durch die entsprechenden Schlitze 65 hindurchfahren, werden sie mit dem heissschmelzenden Adhesiv besprüht,

EP 0 095 034 B2

das durch die Einlassbohrung 56 aus dem Behälter austritt und seinen Weg durch den Verteilkanal 57, durch Düsenöffnungen 59 und die verlängerte Ausnehmung 58 zu den jeweiligen Schlitzen 65 nimmt. Eine züberschüssige Menge diese heiss schmelzenden Klebemittels, das nicht auf das elastiche Teil 15 aufgetragen wird, wird in einem Behälter (nicht dargestellt), der unter der Düse 42 angeordnet ist, wieder aufgefangen.

Vorzugsweise wird angestrebt, dass das elastische Teil mit einer genau bemessenen Menge dieses heiss schmelzenden Adhesivs bei einer geeigneten Temperatur versehen wird. Von den Erfindern durchgeführte Versuche haben gezeigt, dass die bereits erwähnten Naturgummifäden 15A mit einem Querschnitt von annähernd 0,185 mm$^2$, die von der Firma Rondex Co. Ltd. unter dem Namen ≪Rondex 3 SR≫ vertrieben werden, ein bevorzugtes Produkt zur Verwendung als elastisches Teil darstellen. Wenn dieses besondere Produkt als Material für das elastische Teil Verwendung findet, dann beträgt die bevorzugte Menge von heiss schmelzendem Adhesiv, die auf einen solcher Faden 15A aufgetragen wird, zwischen 0,1—0,2 g/m, und die bevorzugte Temperatur zum Auftragen ist eine, die geringer ist als 165°C. Eine Menge von weniger als 0,1 g/m würde verhindern, dass die elastischen Teile 15 sicher an den Seitenklappen 14 der Wegwerfwindeln befestigbar sind, während eine grössere Menge als 0,2 g/m ein Hindurchtreten des Adhesivs durch die Oberlage 12 der Windel 10 bewirken und für den Träger nach Verfestigung zu Unbequemlichkeiten führen würde, ganz abgesehen von der Erhöhung der Herstellkosten. Eine Temperatur von 165°C oder höher würde zu einer Zerstörung oder einem Bruch der Gummifäden 15A führen. Es wurde desweiteren herausgefunden, dass das heiss schmelzende Adhesiv, welches in Japan unter dem Handelsnamen ≪Kanebo NSC MQ-975≫ erhältlich ist, und eine Viskosität von 120 000 cps bzw. 220 000 cps bei einer Temperatur von 120 bzw. 150°C aufweist, eines der bevorzugten Produkte ist.

Die Anzahl der Schlitze 65 hängt ab von der Anzahl der Gummifäden 15, welche in den jeweiligen Seitenklappen 14 der Windel 10 parallel angeordnet werden sollen. Die Abstände, in denen die jeweiligen Schlitze 65 angeordnet sind, dienen dazu, die Abstände zu steuern, in welchen diese Gummifäden 15A in den jeweiligen Seitenklappen 14 der Windel 10 parallel angeordnet werden.

Obwohl das erfindungsgemässe Verfahren wie obenstehend in Verbinfung mit den Fig. 5 bis 7, in welchen die Vorrichtung zum Durchführen dieses erfindungsgemässen Verfahrens dargestellt ist, beschrieben worden ist, wobei jedes elastische Teil 15 eine gewünschte Anzahl von Gummifäden 15A, die parallel zueinander angeordnet sind, in zwei Blöcke 15A, und 15A$_2$ geteilt wird, während die Gummifäden 15A gedehnt werden und dann diese beiden Blöcke 15A$_1$, 15A$_2$ des weiteren jeweils in individuelle Gummifäden 15A aufgeteilt werden, sind andere Ausführungsformen, wie die folgenden, ebenfalls im Bereich des Erfindungsgedanken des Verfahrens enthalten:

a) das elastiche Teil 15, das eine gewünschte Anzahl von Gummifäden 15A aufweist, die parallel aneinanderliegend miteinander verbunden sind, wird in individuelle Gummifäden 15A getrennt, während das elastische Teil 15 einem Zieheffekt ausgesetzt ist;

b) das eine gewüschte Anzahl parallel miteinander verbundener Gummifäden 15A enthaltende elastische Teil 15 wird in zwei Blöcke 15A$_1$ und 15A$_2$ geteilt, nachem es gedehnt worden ist, und anschliessend werden diese beiden Blöcke 15A$_1$ und 15A$_2$ weiter in die individuellen Gummifäden 15A aufgetrennt; und

c) das eine gewüschte Anzahl parallel laufender, miteinander verbundener Gummifäden 15A aufweisende elastische Teil 15 wird nach dem Dehnen in individuelle Gummifäden 15A aufgeteilt.

Vorrichtungen, die so ausgebildet sind, dass sie diese Ausführungsarten a bis c ausführen können, sind in den Zeichnungen nicht dargestallt, können aber von einem Durchschnittsfachmann in Verbindung mit den in den Fig. 5 bis 7 dargestellten Vorrichtungen leicht entworfen werden.

Das elastische Teil 15, das eine Vielzahl von parallel zueinander angeordnete und provisorisch miteinander verbundene Gummifäden 15 aufweist, wird gemäss dem erfindungsgemässen Verfahren verwendet, so wie bereits erwähnt, so dass die einzelnen Gummifäden 15A nicht Gefahr laufen, sich zu verschlingen oder während der Behandlung in der Auftragsstation A und der Ziehstation B zu brechen. Desweiteren werden die Abstände der Gummifäden 15A durch die jeweiligen Schlitze 65 der Düsenmittel 52 gesteuert und die Gummifäden werden auf diese Weise rundherum mit dem Adhesiv versehen, so dass jedes elastiche Teil 15 sicher in jeder Seitenklappe 14 der Windel 10 befestigt ist. Dementsprechend ermöglicht das erfindungsgemässe Verfahren die elastischen Teile 15 wirkungsvoll in die Windel einzuarbeiten, selbst wenn diese elastischen Teile 15 aus einer Vielzahl von extrem dünnen Gummifäden 15A bestehen.

*Beispiel:*

Ein Absorbierkörper bestehend aus einer flockigen Holzpulpe mit einem Einheitsgewicht von 450 g/m$^2$, wurde zwischen der oberen Lage mit den Massen 330 × 450 mm und einem Gewicht von 35 g/m$^2$, bestehend aus 50 Gew.-% Polyesterfaser und 50 Gew.-% Rayonfaser und einer unteren Lage gleicher Abmessung, bestehend aus 25 μ dickem Polyäthylenfilm, eingelegt. Die oberer Lage, die untere Lage und der Absorbierkörper wurden mittels eines heiss schmelzenden Adhesivs miteinander befestigt; die elastischen Teile wurden klebend in beide Seitenklappen eingearbeitet, die durch die Bereiche der beiden Lagen gebildet wurden, welche sich nach aussen über die jeweiligen äusseren Ränder des Absorbierkörpers erstrecken und aufeinanderliegen, so dass eine komplette Windel gemäss Fig. 1 erhalten wurde. Die jeweiligen elastischen Teile bestanden aus drei Naturgummifäden (Gesamtquerschnittfläche 1,32

7

mm²) mit einer Länge von 160 mm, einer Dicke von 0,2 mm und einer Breite von 2,2 mm. Diese drei Gummifäden waren in Abständen zu je 9 mm parallel angeordnet, wobei sie auf eine Länge von 450 mm gestreckt worden sind, so dass eine Zugspannung von 130 g erzielt wurde. Die elastischen Teile waren mit einem heiss schmelzenden Adhesivagens bei einer Temperatur von 160°C aufgeklebt, wobei eine Klebstoffmenge von 0,2 g/m² für jeden Faden verwendet wurde. Das verwendete Adhesivagens hatte eine Viskosität von 20 000 cps/160°C und einen Erweichungspunkt von 97°C.

Als Kontrollprodukt wurde eine Windel hergestellt, in der die Anzahl der Gummifäden unterschiedlich von der oben erwähnten war.

In dieser Kontrollwindel waren elastische Teile mit jeweils einem einzigen Gummiband eingearbeitet, welches 195 mm lang, 0,2 mm dick und 6,6 mm breit war (Querschnittsfläche 1,32 g/m²), welches Band in den jeweiligen Seitenklappen befestigt war und welches gedehnt worden ist, um eine Länge von 450 mm mit einer Zugspannung von annähernd 130 g zu erhalten.

Das efindungsgemässe Produkt und das Kontrollprodukt wurden jeweils 100 Kindern mit einem Gewicht zwischen 5500 und 11 000 g angelegt und bezüglich Urinundichtigkeit und Druckstellen getestet, wobei folgende Resultate erzielt worden sind.

| | Dichtetest | | Druckmarkentest | | |
|---|---|---|---|---|---|
| | keine Undichtheit | leichte Undichtheit | klare Druck-marken | leichte Druck-marken | keine Druck-marken |
| Erfindungsgemässes Produkt | 88 | 12 | 16 | 43 | 41 |
| Kontrollprodukt | 83 | 17 | 42 | 37 | 21 |

Betreffs der Materialmenge, die für das elastische Teil verwendet wurde, war die Anfangsbemessung von 160 mm notwendig, um bei dem erfindungsgemässen Produkt die gewünschte Zugspannung zu erzielen, während die Anfangsbemessung von 195 mm für das Kontrollprodukt erforderlich war, um die gleiche Zugspannung zu erzielen. Es ist aus dem obigen Resultat offensichtlich, das das erfindungsgemässe Produkt wirtschaftliche Vorteile im Vergleich zu dem Kontrollprodukt bietet.

**Patentansprüche**

1. Verfahren zum Einarbeiten bzw. Einlegen von elastischen Teilen in eine Wegwerfwindel bestehend aus einer oberen und einer unteren Lage und einem zwischen diesen beiden Lagen befestigten Körper mit absorbierenden Eigenschaften und mit jeweils durch sich über beide Seiten der einander gegenüberliegenden Außenränder des Absorbierkörpers hinauserstreckende Seitenklappen, zwischen denen jeweils mindestens drei in geeigneten Abständen parallel zueinander über einen Breitenbereich von 10 mm bis 35 mm hinweg Gummifäden gehalten sind, dadurch gekennzeichnet, daß folgende Verfahrensschritte durchgeführt werden:

— Zuführen eines elastischen Bandes, bestehend aus sechs (6) bis neunzig (90) Gummifäden, die parallel verlaufend miteinander verbunden sind, wobei die Gummifäden jeweils im wesentlichen einen Querschnitt von 0,03 mm² bis 0,45 mm² und einen Gesamtquerschnitt von 0,18 mm² bis 2,7 mm² aufweisen;

— Trennen der Gummifäden entlang ihrer Verbindungen in einzelne Gummifäden, während sie in einem Verhältnis von 100% bis 400% gedehnt werden,

— Zuführen von jeweils drei (3) bis fünfundvierzig (45) Gummifäden der sechs (6) bis neunzig (90) Gummifäden zu Bereichen der Windel hin, um in die Seitenklappen in geeigneten Abständen eingeformt zu werden,

— Aufbringen eines adhäsiven Agens auf die Gummifäden,

— Ankleben dieser Gummifäden in gedehntem Zustand in den oben bezeichneten Bereichen unmittelbar auf den jeweiligen Lagen in den Seitenklappen.

2. Verfahren nach Anspruch 1, dadurch gekennzeichnet, dass das elastische Teil bestehend aus sechs (6) bis neunzig (90) parallel zueinander angeordneten, miteinander verbundenen Gummifäden in zwei Blöcke aufgeteilt wird, welche jeweils drei (3) bis fünfundvierzig (45) Gummifäden enthalten, während das elastische Teil gedehnt wird, und dass diese beiden Blöcke desweiteren in einzelne Gummifäden aufgeteilt werden.

3. Verfahren nach Anspruch 1, dadurch gekennzeichnet, dass das elastische Teil in einzelne Gummifäden aufgeteilt wird, während dieses elastische Teil, das sechs (6) bis neunzig (90) parallel angeordnete und miteinander verbundene Gummifäden aufweist, gedehnt wird.

4. Verfahren nach Anspruch 1, dadurch gekennzeichnet, dass das elastische Teil, das sechs (6) bis neunzig (90) Gummifäden aufweist, die parallel zueinander angeordnet und miteinander verbunden sind, in zwei jeweils drei (3) bis fünfundvierzig (45) Gummifäden aufweisende Blöcke geteilt wird, nachdem das

# EP 0 095 034 B2

elastische Teil gedehnt worden ist, und dass diese beiden Blöcke desweiteren in einzelne Gummistränge geteilt werden.

5. Verfahren nach Anspruch 1, dadurch gekennzeichnet, dass das elastische Teil bestehend aus sechs (6) bis neunzig (90) Gummifäden, die parallel zueinander verlaufend miteinander verbunden sind, zunächst gedehnt und anschliessend in einzelne Gummifäden geteilt wird.

6. Verfahren nach mindestens einem der Ansprüche 1 bis 5, dadurch gekennzeichnet, dass die einzelnen getrennten Gummifäden in Abständen voneinander an der Windel befestigt werden, welche Abstände durch die Abstände von Düsenschlitzen bestimmt werden, durch welche hindurch ein Klebeagens auf die einzelnen Gummifäden aufgebracht wird.

7. Verfahren nach Anspruch 1, dadurch gekennzeichnet, dass das elastische Teil, das sechs (6) bis neunzig (90) Gummifäden aufweist, eine innere Trennbarkeit besitzt, jedoch vermittels einer provisorischen Befestigung in Form eines Einzelbandes gehalten wird, wobei diese provisorische Verbindung sauber durch eine leichte Zugkraft in den einzelnen Gummifäden trennbar ist.

## Revendications

1. Procédé d'incorporation d'éléments élastiques dans une couche jetable constituée d'une feuille supérieure et d'une feuille inférieure, et d'un corps absorbant placé entre ces deux feuilles et des rabats latéraux s'étendant par dessus les deux côtés des bords extérieurs opposés du corps absorbant, entre lequels sont maintenus à des distances appropriées parallèlement entre eux au moins trois fils de caoutchouc sur une zone de largeur de 10 mm à 35 mm, caractérisé en ce que l'on exécute les étapes de procédé consistant:

— à fournir des éléments élastiques se présentant sous la forme d'une bande constituée de six (6) à quatre-vingt-dix (90) fils de caoutchouc parallèles jointifs, ces fils présentant des sections individuelles égales de l'ordre de 0,03 à 0,45 mm², et une section totale de 0,18 à 2,7 mm²,

— à séparer ces fils de caoutchouc le long de leurs joints, pour former des fils individuels, tout en les étirant avec un rapport d'étirement de 100 à 400%,

— à amener de trois (3) à quarante-cinq (45) fils de caoutchouc, parmi les six (6) à quatre-vingt-dix (90) disponibles sur les zones de la couche destinées à former les rabats latéraux, en choisissant convenablement les intervalles entre ces fils de caoutchouc,

— dépôt d'un agent adhésif sur les fils de caoutchouc,

— collage de ces fils de caoutchouc à l'état tendu dans les zones ci-dessus mentionnées directement sur les couches respectives dans les rabats latéraux.

2. Procédé selon la revendication 1, caractérisé en ce que l'élément élastique comprenant de six (6) à quatre vingt dix (90) fils de caoutchouc parallèles jointifs, est divisé en deux blocs comprenant chacun de trois (3) à quarante cinq (45) fils de caoutchouc, tandis que cet élément élastique est étiré, les deux blocs étant ensuite séparés en fils de caoutchouc individuels.

3. Procédé selon la revendication 1, caractérisé en ce que l'élément élastique est séparé en fils de caoutchouc individuels pendant l'étirement de cet élément élastique comprenant de six (6) à quatre vingt dix (90) fils de caoutchouc parallèles jointifs.

4. Procédé selon la revendication 1, caractérisé en ce que l'élément élastique comprenant de six (6) à quatre vingt dix (90) fils de caoutchouc parallèles jointifs, est divisé en deux blocs comprenant chacun de trois (3) à quarante cing (45) fils, après étirement de l'élément élastique, ces deux blocs étant ensuite séparés en fils de caoutchouc individuels.

5. Procédé selon la revendication 1, caractérisé en ce que l'élément élastique comprenant de six (6) à quatre vingt dix (90) fils de caoutchouc parallèles jointifs, est étiré puis séparé ensuite en fils de caoutchouc individuels.

6. Procédé selon la revendication 1—5, caractérisé en ce que de caoutchouc individuels séparés sont fixés dans la couche à des intervalles réguliers déterminés par les intervalles des fentes d'ajutage permettant d'appliquer la colle sur les fils de caoutchouc individuels.

7. Procédé selon la revendication 1, caractérisé en ce que les éléments élastiques comprenant de six (6) à quatre vingt dix (90) fils de caoutchouc présentent une possibilité intrinsèque de séparation mais sont maintenus sous la forme d'une bande unique par des joints provisoires pouvant se déchirer nettement sous l'action d'une force faible de façon que l'élément se sépare en fils de caoutchouc individuels.

## Claims

1. "A method of incorporating or inserting elastic members into a disposable diaper including a topsheet and a backsheet as well as an absorbent body, which is fixedly interposed between said both sheets, and side flaps extending beyond both sides of the opposite outer edges of the absorbent body, at least three rubber strings, which are arranged at adequate distances from and parallel to one another and which extend across a width range of from 10 mm to 35 mm, being held between said respective side flaps, characterized in that the following method steps are carried out:

— feeding an elastic tape consisting of six (6) to ninety (90) rubber strings joined together in parallel,

9

said rubber strings each having substantially a cross-section of 0.03 mm² to 0.45 mm² and an overall cross-section of 0.18 mm² to 2.7 mm².

— separating the rubber strings along joints thereof into individual rubber strings while stretching them at a ratio of 100% to 400%,

— supplying respectively three (3) to forty-five (45) rubber strings of said six (6) to ninety (90) rubber strings onto regions of the diaper so as to be formed into the side flaps at adequate intervals.

— applying an adhesive agent to these rubber strings,

— adhesively securing these rubber strings in the stretched condition in the above-mentioned regions directly on the respective sheets in the side flaps."

2. A method as claimed in claim 1, characterized in that the elastic member comprising six (6) to ninety (90) rubber strings joined together in parallel is divided into two blocks each comprising three (3) to forty-five (45) rubber strings while said elastic member is stretched, and then these two blocks are further separated into individual rubber strings.

3. A method as claimed in claim 1, characterized in that the elastic member is separated into individual rubber strings while said elastic member comprising six (6) to ninety (90) rubber strings joined together in parallel is stretched.

4. A method as claimed in claim 1, characterized in that the elastic member comprising six (6) to ninety (90) rubber strings joined together in parallel is divided into two blocks each comprising three (3) to forty-five (45) rubber strings after said elastic member is stretched, and then these two blocks are further separated into individual rubber strings.

5. A method as claimed in claim 1, characterized in that the elastic member comprising six (6) to ninety (90) rubber strings joined together in parallel is stretched and then separated into individual rubber strings.

6. A method as claimed in any one of claims 1 to 5, characterized in that the individually separated rubber strings are secured in the diaper at intervals regulated by intervals of nozzle slots through which said adhesive agent is applied to the individual rubber strings.

7. A method as claimed in any of claim 1, characterized in that the elastic members comprising six (6) to ninety (90) rubber strings having intrinsic separability but maintained in the form of a single tape by provisional joints may be neatly separated by a slight tearing force into individual rubber strings.

# FIG. 1

# FIG. 2

# FIG. 3

(A)    (B)

# FIG. 4

1

EP 0 095 034 B2

# FIG. 5

# FIG. 6

# FIG. 7

# FIG. 8

# FIG. 9

# FIG. 10

# FIG. 11

# FIG. 12